# EUROPÄISCHE PATENTANMELDUNG

(11) **EP 4 635 937 A1**
(43) Veröffentlichungstag der Anmeldung: **22.10.2025**
(21) Anmeldenummer: 24170950.0
(22) Anmeldetag: 18.04.2024
(51) Int. Cl.: C07C 45/45, C07C 49/08, C07C 1/20, C07C 11/06, C07C 11/09, C07C 11/167, C12P 7/06, C12P 7/28

(54) **VERFAHREN ZUR HERSTELLUNG MINDESTENS EINES FUNKTIONALISIERTEN NIEDERMOLEKULAREN KOHLENWASSERSTOFFS**

(71) Anmelder: Evonik Operations GmbH, 45128 Essen (DE)
(72) Erfinder: METTERNICH, Jan Benedikt, 45657 Recklinghausen (DE); NITZ, Jörg-Joachim, 45257 Essen (DE); ZANTHOFF, Horst-Werner, 45481 Mülheim a.d. Ruhr (DE); KREIS, Peter, 44227 Dortmund (DE); BECK, Simon, 48161 Münster (DE); HILLER, Christoph, 48249 Dülmen (DE); SCHALLENBERG, Jörg, 46286 Dorsten (DE); BLÜMKE, Wilfried, 61137 Schöneck (DE); KOHLSTRUK, Stephan, 45966 Gladbeck (DE)
(74) Vertreter: Evonik Patent Association

(57) **Zusammenfassung**

Gegenstand der vorliegenden Erfindung ist ein Verfahren zur chemischen Umsetzung (I) von Ethanol und Wasser zu mindestens einem funktionalisierten niedermolekularen Kohlenwasserstoff K, Kohlendioxid und Wasserstoff, bei dem mindestens ein Teil des entstehenden Kohlendioxids und/oder Wasserstoffs aus dem Produktgemisch abgetrennt (II) und einem fermentativen Reaktionsschritt (III) zugeführt wird, mit dem Ethanol und/oder mindestens ein funktionalisierter niedermolekularer Kohlenwasserstoff K hergestellt wird, und nachfolgend ggf. entstandenes Ethanol der chemischen Umsetzung (I) zugeführt wird und ggf. entstandener funktionalisierter niedermolekularer Kohlenwasserstoff K mit dem Produktstrom der chemischen Umsetzung (I) vereinigt wird.

## Beschreibung

Die vorliegende Erfindung betrifft ein Verfahren zur Herstellung mindestens eines funktionalisierten niedermolekularen Kohlenwasserstoffs. Insbesondere betrifft die vorliegende Erfindung ein Verfahren zur Herstellung mindestens eines funktionalisierten Kohlenwasserstoffs, der zwei bis vier Kohlenstoffatome aufweist. Noch weiter bevorzugt betrifft die vorliegende Erfindung ein Verfahren zur Herstellung mindestens eines funktionalisierten Kohlenwasserstoffs mit zwei bis vier Kohlenstoffatomen aus Ethanol in der Gegenwart von Wasser.

Funktionalisierte niedermolekulare Kohlenwasserstoffe sind lineare, verzweigte oder cyclische Verbindungen mit bis zu 10 Kohlenwasserstoffatomen, die mindestens eine Funktionalisierung aufweisen. Unter mindestens einer Funktionalisierung ist dabei das Vorliegen mindestens einer funktionellen Gruppe zu verstehen, die eine Umsetzung des funktionalisierten Kohlenwasserstoffs zu anderen, ggf. ebenfalls funktionalisierten Kohlenwasserstoffen, insbesondere solchen mit höherem Molekulargewicht, ermöglicht. Bevorzugte funktionelle Gruppen sind Kohlenstoff-Kohlenstoff-Doppelbindungen, Kohlenstoff-Kohlenstoff-Dreifachbindungen und an Kohlenstoffatome der Kohlenwasserstoffe gebundene Hydroxy- und Carbonylgruppen.

Funktionalisierte niedere Kohlenwasserstoffe wie Butadien, Isobuten, Propylen und Aceton sind für industrielle und chemische Anwendungen von großem Interesse. Butadien ist von großer Bedeutung u.a. für die Herstellung von Synthesekautschuk, Acrylnitril-Butadien-Styrol-Copolymeren und Adiponitril. Isobuten, auch bekannt als Isobutylen oder 2-Methylpropen, ist ein Kohlenwasserstoff von erheblichem Interesse, der häufig als Zwischenprodukt bei der Herstellung industriell wichtiger Produkte verwendet wird. Zu nennen sind hier insbesondere Treibstoffe, Benzinoxygenate, Isooctan, Methacrolein, Methylmethacrylat und Butylkautschuk. Propylen, auch Methylethylen oder Propen genannt, ist ein Kohlenwasserstoff von erheblichem Interesse, der häufig als Zwischenprodukt bei der Herstellung von Kunststoff-Polypropylen verwendet wird, das in der gesamten Industrie bei der Herstellung von Folien, Verpackungen, Kappen und Verschlüssen verwendet wird. Weiterhin ist Propen ein wichtiges Edukt für die Herstellung von Acrylsäure, Acrylaten und Acrylamiden, die Anwendung finden bei der Herstellung von Superabsorbern. Aceton ist ein Kohlenwasserstoff von erheblichem Interesse, der häufig als Zwischenprodukt bei der Herstellung industriell wichtiger Produkte, insbesondere für die Synthese von Isophoron, Isophorondiamin, Methylmethacrylat und Bisphenol A, sowie als Lösungsmittel für Reinigungszwecke verwendet wird.

In der Vergangenheit wurden funktionalisierte niedere Kohlenwasserstoffe zunächst über die Trockendestillation von Holz erhalten. Bereits in den 30er Jahren des letzten Jahrhunderts wurde als eine Alternative die Synthese der funktionalisierten Kohlenwasserstoffe aus Ethanol in Erwägung gezogen (Synthese von Aceton aus Ethanol, US 1,978,619 A). Schlussendlich war es für eine lange Zeit jedoch wirtschaftlicher, funktionalisierte Kohlenwasserstoffe aus Erdöl bzw. seinen Folgeprodukten herzustellen.

Aufgrund der bekannten Nachteile der Nutzung fossiler Rohstoffe (u.a. wegen des hohen COz-Fußabdrucks) werden seit einiger Zeit nun jedoch wieder alternative Wege erforscht, funktionalisierte Kohlenwasserstoffe zugänglich zu machen.

Von großer Relevanz ist dabei die Synthese funktionalisierter Kohlenwasserstoffe aus Ethanol, der u.a. über Gärprozesse aus Kohlenhydraten zugänglich ist. Die Synthese des erforderlichen Ethanols kann auch aus Biomasse-generiertem Synthesegas erfolgen, wie z. B. in WO 02/08438 A2 offenbart. Auch US 2012/0052541 A1 und US 2010/0317074 A1 offenbaren Verfahren, bei denen u.a. Ethanol fermentativ aus Synthesegas hergestellt werden kann. U.a. US 2012/0052541 A1 lehrt, dass nicht umgesetzte Edukte und Nebenprodukte der Fermentation dem Fermentationsprozess wieder zugeführt werden können.

Ähnlich wie bei dem großindustriell eingesetzten Verfahren der Dampfreformierung von Methan (1) kann ggf. fermentativ erhaltenes Ethanol (2) in Gegenwart von Wasser zu einem Oxid des Kohlenstoffs und H₂ umgesetzt werden (Can J Chem Eng. 2023, 101 :5498-5518). Über das Boudouard-Gleichgewicht (3) lassen sich dabei entstandenes Kohlenmonoxid in Kohlenstoff und Kohlendioxid umwandeln und umgekehrt:

CH₄ + H₂O → CO + 3 H₂ (1)

C₂H₅OH + 3 H₂O → 6 H₂ + 2 COz (2)

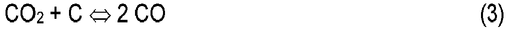

Der Stand der Technik offenbart jedoch auch Verfahren, mit denen bei Verwendung geeigneter Katalysatoren alternativ auch für die chemische Industrie hochinteressante Verbindungen zugänglich gemacht werden können. Diese sind von besonderem Interesse für die Synthesechemie. Von ganz besonderem Interesse ist dabei die Umsetzung von Ethanol zu Acetaldehyd, Aceton, Propen, Isobuten oder Butadien.

US 1,978,619 A offenbart ein Verfahren zur Herstellung von Aceton, bei dem Ethanol in Gegenwart von Schwermetalloxid-Katalysatoren bei Temperaturen von 250 - 650 °C umgesetzt wird.

J. Chem. Soc., Chem. Commun., 1987, 394-395 offenbart, dass Ethanol in Gegenwart von H₂O und Katalysatoren wie ZnO-Cr₂O₃, Fe₂O₃-CaO und ZnO-Cr₂O₃-K₂O zu Aceton (und geringen Anteilen von Methan, Propen und Isobuten) umgesetzt werden kann.

Journal of Catalysis 109, 298-302 (1988) offenbart, dass Ethanol in Gegenwart von H₂O und Katalysatoren wie Fe₂O₃:CaO, Fe₂O₃:MnO und Fe₂O₃:ZnO über das Zwischenprodukt Acetaldehyd zu Aceton umgesetzt werden kann.

Auch Appl. Catal. 1989, 52(3), 237-248 offenbart die Umwandlung von Ethanol zu Aceton in der Gegenwart von Wasserdampf. Hier wird als Katalysator ZnO-CaO eingesetzt. Auch hier wird angenommen, dass zwischenzeitlich Acetaldehyd gebildet wird.

J. Mater. Chem. 1994, 4(6), 853-858 befasst sich mit einem Screening von 24 Oxiden bei der Umwandlung von Ethanol in Aceton in Gegenwart von HzO. Dabei wurde festgestellt, dass Eisen-, Mangan- und Zink-Katalysatoren besonders gut geeignet sind. Der optimale Katalysator war ein Fe₂O₃-ZnO Katalysator vom Spinell-Typ.

Appl. Catal. A: General 2013, 458, 111-118 betrachtet Ethanol als Plattform-Molekül und postuliert Mechanismen, bei denen das bei der Umsetzung von Ethanol mit Wasser zunächst gebildete Acetaldehyd entweder zum 1-Buten oder zum Aceton umgesetzt werden könne. Die in dieser Studie verwendeten Katalysatoren (Cu/ZnO/Al₂O₃ und ZrOz) seien besonders geeignet für die Umsetzung zu Aceton.

Catal. Sci. Technol. 2021, 11(6), 2047-2056 befasst sich mit der Bildung von Aceton bzw. Isobuten aus Ethanol in Gegenwart von Wasserdampf. Es werden Ga₂O₃-ZrO₂-Katalysatoren eingesetzt. Es wird angenommen, dass das gebildete Isobuten aus Zersetzung eines aus zwei Acetonmolekülen über Aldoladdition gebildeten Mesityloxid-Intermediats zu Isobuten und Essigsäure entsteht. Die Essigsäure würde nachfolgend in Aceton und COz umgewandelt.

ACS Catalysis 2022, 12(8), 4358-4374 stellt heraus, dass die Reaktionsmechanismen bei der Umwandlung von Ethanol noch nicht vollständig verstanden sind. Ein Beispiel hierfür sei die Umsetzung von Ethanol mit Wasser zu verschiedenen Produkten in Gegenwart eines Katalysators, die als Dampfreformierung von Ethanol bezeichnet wird. Die hier betrachtete Verfahrensvariante führt zu Aceton und Crotonaldehyd. Crotonaldehyde sind ungesättigte Aldehyde mit vier Kohlenstoffatomen und einer Aldehydgruppe. Es wird ausgeführt, dass bei der Bildung von Aceton über C-C-Kopplung ein C4-Intermediat gebildet werde, von dem im Nachgang CO bzw. COz abgespalten werde. Aus diesem Grund sei COz ein oft auftretendes Begleitprodukt. In der vorliegenden Studie wird beobachtet, dass die Anwesenheit von Wasser das Reaktionsgleichgewicht in Richtung vermehrter Acetonproduktion (und verminderter Crotonaldehydbildung) verschiebt.

WO 2016/061262 A1 offenbart ein Verfahren zur Bildung funktionalisierter niederer Kohlenwasserstoffe, bei dem Ethanol über einem gemischten Metalloxid-Katalysator mit Wasser umgesetzt wird. Bevorzugt lassen sich mit diesem Verfahren Isobutylen, Propylen und Aceton herstellen.

Die bislang bekannten chemischen Verfahren zur Umsetzung von Ethanol und Wasser zu mindestens einem funktionalisierten niedermolekularen Kohlenwasserstoff haben gemein, dass bei ihnen u.a. Gase, insbesondere COz und H₂, entstehen, die im Fall von COz zum Treibhauseffekt beitragen und zu einer Verminderung der Ausbeute führen.

Die Aufgabe der vorliegenden Erfindung ist es somit, die bestehenden Nachteile des Standes der Technik zu überwinden. Insbesondere ist es die Aufgabe der vorliegenden Erfindung, ein chemisches Verfahren zur Umsetzung von Ethanol und Wasser zu mindestens einem funktionalisierten niedermolekularen Kohlenwasserstoff bereitzustellen, das weniger Treibhausgase emittiert und zu höheren Ausbeuten führt.

Die sich vorliegend stellende Ausgabe wird gelöst durch das erfindungsgemäße Verfahren zur chemischen Umsetzung (I)
- von Ethanol und Wasser
- zu mindestens einem funktionalisierten niedermolekularen Kohlenwasserstoff K, Kohlendioxid und Wasserstoff,
bei dem
mindestens ein Teil des entstehenden Kohlendioxids und/oder Wasserstoffs von dem Produktgemisch
- abgetrennt (II) und
- einem fermentativen Reaktionsschritt (III) zugeführt wird, mit dem Ethanol und/oder mindestens ein funktionalisierter niedermolekularer Kohlenwasserstoff K hergestellt wird und nachfolgend
- ggf. entstandenes Ethanol der chemischen Umsetzung (I) zugeführt wird und
- ggf. entstandener funktionalisierter niedermolekularer Kohlenwasserstoff K mit dem Produktstrom der chemischen Umsetzung (I) vereinigt wird.

Anschließend an die chemische Umsetzung (I) wird in Schritt (II) mindestens ein Teil des entstehenden Kohlendioxids und/oder Wasserstoffs aus dem Produktgemisch abgetrennt.

### Abtrennung (II)

Bevorzugt wird mindestens ein Teil des entstehenden Kohlendioxids und Wasserstoffs von dem Produktgemisch abgetrennt. Verfahren zur Abtrennung von Kohlendioxid und/oder Wasserstoff von Produktgemischen sind prinzipiell bekannt.

Bevorzugt kann die Abtrennung (II) des entstehenden gasförmigen Kohlendioxids und/oder Wasserstoffs von dem Produktgemisch mit Hilfe einer Kondensation durch Temperaturabsenkung/Kühlung herbeigeführt werden. Dabei kondensiert größtenteils der niedermolekulare Kohlenwasserstoff K, ggf. nicht umgesetztes Ethanol, Wasser und etwaige Nebenprodukte aus, während Kohlendioxid und Wasserstoff, in der gasförmigen Phase verbleiben. Bevorzugt wird dabei mindestens ein Wärmetauscher eingesetzt. Weiter bevorzugt werden in der technischen Anwendung zwei, drei oder mehr, bevorzugt zwei bis fünf Wärmetauscher in einer Kaskade verwendet. Die Ausführung ist hierbei abhängig von der jeweiligen stofflichen Zusammensetzung, des Druckes und der zur Verfügung stehenden Betriebsmittel, welche zur Kühlung eingesetzt werden.

Bevorzugt wird dabei die Abtrennung mittels Kondensation in einer ersten Kondensations-/Kühlungsstufe bei Temperaturen von -50 °C bis 180 °C und bei dem Druck des Reaktionsteils durchgeführt. So kann in der nicht kondensierten Gasphase der Wassergehalt auf 0,5 bis 20 Gew.-% und der Anteil an Kohlenwasserstoffen auf 0,5 bis 25 Gew.-% reduziert werden. Noch weiter bevorzugt wird die Abtrennung bei Temperaturen von 30 °C bis 120°C und bei dem Druck des Reaktionsteils durchgeführt. So kann in der nicht kondensierten Gasphase der Wassergehalt auf 1 bis 10 Gew.-% und der Anteil an Kohlenwasserstoffen auf 1 bis 15 Gew.-% reduziert werden.

Um eine möglichst hohe Ausbeute bei der Trennung zu erreichen, kann nach einer ersten Kondensation der Druck des nicht kondensierten Anteils des Dampf-Gas-Gemisches mit Hilfe einer Kompressionsstufe oder einer mehrstufigen Verdichtung erhöht werden. Bevorzugt wird der Druck auf 1 bis 30 bar, weiter bevorzugt auf 3 bis 10 bar erhöht.

Bei höherem Druck kann das Gas-Dampf-Gemisch erneut mit Hilfe einer Kondensation unter Verwendung eines Wärmetauschers gekühlt werden, um das Produktgemisch weiter aufzutrennen. Die zweite Kondensationsstufe bei höherem Druck kann wiederum in einer Kaskade von Wärmetauschern ausgeführt werden. Bevorzugt wird die Abtrennung in einer zweiten Kondensations-/Kühlungsstufe bei Temperaturen von -50 °C bis 80 °C durchgeführt. So kann in der nicht kondensierten Gasphase der Anteil an Kohlenwasserstoffen auf 0,1 bis 10 Gew.-% reduziert werden. Noch weiter bevorzugt wird die Abtrennung in einer zweiten Kondensations-/Kühlungsstufe bei Temperaturen von -40 °C bis 50°C durchgeführt. So kann in der nicht kondensierten Gasphase der Anteil an Kohlenwasserstoffen auf unter 10 Gew.-% reduziert werden.

Besonders gute Ergebnisse, insbesondere in Bezug auf Ausbeutenerhöhung, die Verminderung des COz-Ausstoßes, aber auch in Bezug auf eine besonders einfache Verfahrensführung, werden erzielt, wenn die Abtrennung (II) mit mindestens einem Membranseparationsverfahren erfolgt. Besonders bevorzugt setzt die Abtrennung (II) somit mindestens ein Membranseparationsverfahren ein. Weiter bevorzugt setzt die Abtrennung (II) ein Membranseparationsverfahren ein.

Unter einem Membranseparationsverfahren ist vorliegend ein Trennverfahren zu verstehen, das auf dem unterschiedlichen Transport unterschiedlicher Stoffe durch permeable Membranen beruht.

Bevorzugt liegt aus dem chemischen Umsetzungsschritt (I) stammendes Kohlendioxid und Wasserstoff, weiter bevorzugt das gesamte Produktgemisch des chemischen Umsetzungsschrittes (I), gasförmig vor. Es ist allgemein bekannt, dass Gasgemische aufgrund unterschiedlicher Permeabilitäten der einzelnen Gase mittels Gastrennmembranen getrennt werden können. Besonders geeignet zur Abtrennung von Kohlendioxid und Wasserstoff sind Membranseparationsverfahren, die mindestens eine polymere Membran einsetzen. Entsprechende polymere Gastrennmembranen beruhen bevorzugt auf zu Hohlfasern oder flachen Membranen verarbeiteten Polymeren. Entsprechende Membranen zeichnen sich durch eine sehr dünne aktive Trennschicht aus, so dass die Permeanz der Membran möglichst groß ist.

Bevorzugte polymere Membranen können ausgewählt werden aus der Gruppe bestehend aus glasartigen und gummiartigen Membranen. Bevorzugt wird mindestens eine glasartige Membran eingesetzt. Sie trennen Gase aufgrund der Unterschiede in deren Molekülgröße. Kleine Moleküle wie z.B. H₂, He und H₂O permeieren schneller durch die Polymermatrix im Vergleich zu größeren Molekülen wie CH₄ oder N₂. Bevorzugt umfasst die mindestens eine glasartige Membran mindestens ein Polymer ausgewählt aus der Gruppe bestehend aus Polysulfon (PS), Polyethersulfon (PES), Polyetherimid (PEI), Polyimid (PI), Polyphenylenoxid (PPO), Celluloseacetat (CA) und Fluorpolymeren. Beispielhaft sind hier Air Products PRISM, Evonik SEPURAN und Air Liquide MEDAL genannt.

Ganz besonders bevorzugt wird ein Membranseparationsverfahren eingesetzt, bei dem mindestens eine gummiartige Membran verwendet wird. Gummiartige Membranen lassen leicht kondensierbare Moleküle (HzO, Aceton, VOCs) bevorzugt durch die Membran permeieren und haben demnach geringere Permeabilitäten für permanente Gase wie Stickstoff, Sauerstoff und Wasserstoff. Bevorzugt werden gummiartige Membranen umfassend Polymere ausgewählt aus Silikonen, Polydimethylsiloxanen (PDMS), Polyoctylmethylsiloxanen (POMS), Silikonacrylaten und Polyether-basierten Blockcopolymeren (insbesondere Polyethylenoxid-Block-Amiden) eingesetzt. Beispielhaft sind hier MTR Polaris, Evonik PURAMEM VOC, GMT Membrantechnik oder HZG PolyActive genannt.

Noch weiter wird die Abtrennung (II) so durchgeführt, dass zunächst i) mindestens ein Kondensationsschritt unter Erhalt eines kondensierten Kohlendioxid- und Wasserstoff-armen Produktgemischs und einer Kohlendioxid- und Wasserstoff-reichen gasförmigen Phase durchgeführt wird, und ii) dann die erhaltene gasförmige Phase mit mindestens einem Membranseparationsverfahren unter Erhalt eines gereinigten gasförmigen Kohlendioxid-Wasserstoff-Gemischs aufgereinigt wird.

Weiter bevorzugt kann die Abtrennung des entstehenden Kohlendioxids und Wasserstoffs aus dem Produktgemisch in Teilschritt (II) i) mit Hilfe einer Kondensation durch Temperaturabsenkung /Kühlung unter Verwendung von Wärmetauschern herbeigeführt werden. Bevorzugt wird dabei mindestens ein Wärmetauscher eingesetzt. Weiter bevorzugt werden in der technischen Anwendung häufig zwei, drei oder mehr, bevorzugt zwei bis fünf Wärmetauscher in einer Kaskade verwendet. Die Ausführung ist hierbei abhängig von der jeweiligen stofflichen Zusammensetzung, des Druckes und der zur Verfügung stehenden Betriebsmittel, welche zur Kühlung eingesetzt werden.

Noch weiter bevorzugt wird dabei die Abtrennung mittels Kondensation in einer ersten Kondensation- /Kühlungsstufe bei Temperaturen von -50 °C bis 180 °C und bei dem Druck des Reaktionsteils durchgeführt. So kann in der nicht kondensierten Gasphase der Wassergehalt auf 0,5 bis 20 Gew.-% und der Anteil an Kohlenwasserstoffen auf 0,5 bis 25 Gew.-% reduziert wird. Noch weiter bevorzugt wird die Abtrennung bei Temperaturen von 30 °C bis 120°C und bei dem Druck des Reaktionsteils durchgeführt. So kann in der nicht kondensierten Gasphase der Wassergehalt auf 1 bis 10 Gew.-% und der Anteil an Kohlenwasserstoffen auf 1 bis 15 Gew.-% reduziert werden.

Um eine möglichst hohe Ausbeute bei der Trennung zu erreichen, kann nach einer ersten Kondensation der Druck des nicht kondensierten Anteils des Dampf-Gas-Gemisches mit Hilfe einer Kompressionsstufe oder einer mehrstufigen Verdichtung erhöht werden. Bevorzugt wird der Druck auf 1 bar bis 30 bar, weiter bevorzugt auf 3 bis 10 bar erhöht.

Bei höherem Druck kann das Gas-Dampf-Gemisch erneut mit Hilfe einer Kondensation unter Verwendung eines Wärmetauschers gekühlt werden, um das nicht-kondensierte Produktgemisch weiter aufzutrennen. Die zweite Kondensationsstufe bei höherem Druck kann wiederum in einer Kaskade von Wärmetauschern ausgeführt werden. Bevorzugt wird die Abtrennung in einer zweiten Kondensations-/Kühlungsstufe bei Temperaturen von -50 °C bis 80 °C durchgeführt. So kann in der nicht kondensierten Gasphase der Anteil an Kohlenwasserstoffen auf 0,1 bis 10 Gew.-% reduziert werden. Noch weiter bevorzugt wird die Abtrennung in einer zweiten Kondensations-/Kühlungsstufe bei Temperaturen von - 40 °C bis 50°C durchgeführt. So kann in der nicht kondensierten Gasphase der Anteil an Kohlenwasserstoffen auf unter 10 Gew.-% reduziert werden.

Anschließend wird in Schritt (II) ii) die erhaltene gasförmige Phase auf eine Membrantrennung gegeben, um das Produktgemisch weiter aufzutrennen. Für dieses sich anschließende Membranseparationsverfahren können bevorzugt glasartige und/oder gummiartige Membranen eingesetzt werden. Besonders bevorzugt werden gummiartige Membranen eingesetzt.

Prinzipiell kann die Membrantrennung so erfolgen, dass nur eine Membran eingesetzt wird (1-stufige Membrantrennung). Insbesondere in der großtechnischen Anwendung und/oder zur Erzielung besonders guter Ergebnisse können mehrere Membranen in einer Kaskade verschaltet werden. Besonders bevorzugt sind 2-stufige, 3-stufige und 4-stufige Verschaltungen, d. h., es werden zwei, drei oder vier Membranen in einer Membrankaskade (ggf. auch mit Rückführungen) verschaltet, um Ausbeuten und/oder Reinheiten der Trennstufe zu erhöhen.

Bevorzugt liegen die Druckverhältnisse in der Membrantrennung, d.h. der Quotient aus Feeddruck und Permeatdruck, in einem Bereich von 2 - 300, bevorzugt 3 -100 und besonders bevorzugt 4 - 50.

Bevorzugt liegt der Stage Cut einer einzelnen Membrantrennung, d.h. der Quotient aus Permeat- und Feednormvolumenstrom, zwischen 10% und 80%, bevorzugt zwischen 20% und 60%.

Mit dem beschriebenen Membranverfahren kann der Anteil an Kohlenwasserstoffen in der nicht kondensierten Gasphase durch die Trennsequenz aus Verdichter, Kondensation und Membrantrennung auf 0,1 bis 2 Gew.-% reduziert werden.

In einer weiteren bevorzugten Ausführungsformen kann das Permeat vor eine der Kondensationen zurückgeführt werden, um die Prozesseffizienz weiter zu verbessern.

### Chemische Umsetzung (I)

Das erfindungsgemäße Verfahren ist ein Verfahren zur chemischen Umsetzung (I) von Ethanol und Wasser zu mindestens einem funktionalisierten niedermolekularen Kohlenwasserstoff K, Kohlendioxid (COz) und Wasserstoff (H₂).

Unter einem funktionalisierten niedermolekularen Kohlenwasserstoff K ist dabei eine lineare, verzweigte oder cyclische Verbindung mit bis zu 10 Kohlenwasserstoffatomen zu verstehen, die mindestens eine Funktionalisierung aufweisen. Unter mindestens einer Funktionalisierung ist dabei das Vorliegen mindestens einer funktionellen Gruppe zu verstehen, die eine Umsetzung des funktionalisierten Kohlenwasserstoffs zu anderen, ggf. ebenfalls funktionalisierten Kohlenwasserstoffen, insbesondere solchen mit höherem Molekulargewicht, ermöglicht. Bevorzugte funktionelle Gruppen sind Kohlenstoff-Kohlenstoff-Doppelbindungen, Kohlenstoff-Kohlenstoff-Dreifachbindungen und an Kohlenstoffatome der Kohlenwasserstoffe gebundene Hydroxy- und Carbonylgruppen. Bevorzugt handelt es sich somit bei dem mindestens einen funktionalisierten niedermolekularen Kohlenwasserstoff K um mindestens einen niedermolekularen Kohlenwasserstoff K mit mindestens einer funktionellen Gruppe ausgewählt aus der Gruppe bestehend aus Kohlenstoff-Kohlenstoff-Doppelbindungen, Kohlenstoff-Kohlenstoff-Dreifachbindungen und an Kohlenstoffatome der Kohlenwasserstoffe gebundene Hydroxy- und Carbonylgruppen. Bevorzugt weisen der mindestens eine niedermolekulare Kohlenwasserstoff K zwei bis vier Kohlenstoffatome auf. Noch weiter bevorzugt umfasst der mindestens eine niedermolekulare Kohlenwasserstoff K mindestens eine Verbindung ausgewählt aus der Gruppe bestehend aus Acetaldehyd, Aceton, Propen, Isobuten und Butadien. Darüber hinaus noch weiter bevorzugt umfasst der mindestens eine Kohlenwasserstoff K Aceton und mindestens eine weitere Verbindung ausgewählt aus der Gruppe bestehend aus Acetaldehyd, Propen, Isobuten und Butadien.

Ganz besonders bevorzugt führt das Verfahren zur chemischen Umsetzung (I) von Ethanol und Wasser zu einem funktionalisierten Kohlenwasserstoff K, Kohlendioxid und Wasserstoff. Noch weiter bevorzugt ist der funktionalisierte Kohlenwasserstoff Aceton, d. h. noch weiter bevorzugt ist das erfindungsgemäße Verfahren ein Verfahren, bei dem Ethanol und Wasser zu Aceton, Kohlendioxid und Wasserstoff umgesetzt wird.

Weiter bevorzugt ist das erfindungsgemäße Verfahren ein einstufiges Verfahren zur chemischen Umsetzung (I) von Ethanol und Wasser zu Aceton, Kohlendioxid und Wasserstoff, bei dem Ethanol und Wasser in Gegenwart eines heterogenen Katalysators umfassend mindestens ein aktives Metall ausgewählt aus Zn, Co, Ca, Fe, Mg, Cu, Ga, Cr, Na, K, Zr, Ag, Ce, W, Al, V, La und Sr umgesetzt wird. Bevorzugte Reaktionstemperaturen liegen bei 350 - 500 °C. Bevorzugte Drücke liegen bei 0.5 - 10 bar, bevorzugt 0.5 - 5 bar, noch weiter bevorzugt 0.5 - 2 bar.

Ebenfalls bevorzugt ist das erfindungsgemäße Verfahren ein zweistufiges Verfahren zur chemischen Umsetzung (I) von Ethanol und Wasser zu Aceton, Kohlendioxid und Wasserstoff, bei dem Ethanol zunächst in einem ersten Schritt
i) unter Abwesenheit von Wasser und in Gegenwart eines heterogenen Katalysators umfassend mindestens ein aktives Metall ausgewählt aus Au, Cu, Cr, Zn, Rb, Mg, Al, Fe, and V zu einem Intermediat ausgewählt aus Acetaldehyd, Crotonaldehyd, Essigsäure und Ethylacetat umgesetzt wird und dann
ii) in einem zweiten Reaktionsschritt die Umsetzung des zwischenzeitlich entstandenen Intermediats zu Aceton in Gegenwart von Wasser und einem heterogenen Katalysator umfassend mindestens ein Metall ausgewählt aus Cu, Al, Zn, and Ba erfolgt.

Weiter bevorzugt handelt es sich bei dem Intermediat um Acetaldehyd. Bevorzugte Reaktionstemperaturen für den ersten Reaktionsschritt liegen bei 200 - 300 °C. Bevorzugte Reaktionstemperaturen für den zweiten Reaktionsschritt liegen bei 250 - 450 °C. Bevorzugte Drücke liegen bei 0.5 - 10 bar, bevorzugt 0.5 - 5 bar, noch weiter bevorzugt 0.5 - 2 bar.

Besonders gute Ergebnisse resultieren, wenn das molare Verhältnis von Wasser zu Ethanol im Bereich von 0,5 : 1 bis 10 : 1 liegt.

Zur Erzielung vorteilhafter Eigenschaften kann die Umsetzung weiterhin bevorzugt in der Gegenwart von Stickstoff durchgeführt werden. Weiter bevorzugt wird dabei mit Stickstoff in einer Menge von bis zu 90 Vol.-% beaufschlagt.

In Schritt (II) wird anschließend mindestens ein Teil des entstehenden Kohlendioxids und/oder Wasserstoffs abgetrennt. Bevorzugt wird mindestens ein Teil des entstehenden Kohlendioxids und Wasserstoffs abgetrennt. Weiter bevorzugt wird mindestens 90 Gew.-% des gebildeten Kohlendioxids und 90 Gew.-% des gebildeten Wasserstoffs abgetrennt.

Entstehen bei der chemischen Umsetzung (I) nicht funktionalisierte Kohlenwasserstoffe als Nebenprodukt, können diese weiterhin an geeigneter Stelle über Purge-Ströme ausgeschleust werden.

### Fermentativer Reaktionsschritt (III)

Abgetrenntes Kohlendioxid und/oder abgetrennter Wasserstoff, bevorzugt abgetrenntes Kohlendioxid und abgetrennter Wasserstoff, wird nach der Abtrennung einem fermentativen Reaktionsschritt (III) zugeführt, mit dem Ethanol und/oder mindestens ein funktionalisierter niedermolekularer Kohlenwasserstoff K hergestellt wird. Der Feed-Strom des fermentativen Reaktionsschrittes (III) kann ausschließlich aus dem entstandenen Kohlendioxid und/oder Wasserstoff aus dem Produktgemisch des Reaktionsschrittes (I), bevorzugt dem abgetrennten Kohlendioxid und dem abgetrennten Wasserstoff des Reaktionsschrittes (I), bestehen.

Bevorzugt besteht der Feed-Strom des fermentativen Reaktionsschrittes (III) jedoch
a) aus dem in Schritt (II) abgetrennten Kohlendioxid und/oder Wasserstoff aus dem Produktgemisch des Reaktionsschrittes (I), bevorzugt dem abgetrennten Kohlendioxid und dem abgetrennten Wasserstoff des Reaktionsschrittes (I), und
b) Kohlendioxid und/oder Kohlenmonoxid aus anderen Produktionsquellen, ganz besonders bevorzugt aus den Abgasen anderer (insbesondere industrieller) Prozesse,
da dies nicht nur vorteilhaft im Sinne einer Verminderung des COz/CO-Ausstoßes ist, sondern auch die Ausbeuten des fermentativen Reaktionsschrittes (III) erhöht werden können.

Wird Kohlendioxid (COz) aus anderen Produktionsquellen zugesetzt, wird dies bevorzugt in einer Menge von bis zu 10 Vol-% (42 Gew.-%), bezogen auf die eingesetzte Menge an Wasserstoff und Kohlendioxid aus dem chemischen Umsetzungsschritt (I) zugesetzt. Wird Kohlenmonoxid (CO) aus anderen Produktionsquellen zugesetzt, wird dies bevorzugt in einer Menge von bis zu 16 Vol-% (43 Gew.-%), bezogen auf die eingesetzte Menge an Wasserstoff und Kohlendioxid aus dem chemischen Umsetzungsschritt (I) zugesetzt.

Bei dem fermentativen Reaktionsschritt (III) erfolgt die Umsetzung von Kohlendioxid und/oder Wasserstoff zu Ethanol und/oder mindestens einem funktionalisierten niedermolekularen Kohlenwasserstoff K aufgrund der Anwesenheit von Bakterienkulturen, Pilzkulturen, sonstigen biologischen Kulturen oder Enzymen. Bevorzugt erfolgt die Umsetzung von Kohlendioxid und/oder Wasserstoff zu Ethanol und/oder mindestens einem funktionalisierten niedermolekularen Kohlenwasserstoff K aufgrund der Anwesenheit von Bakterienkulturen.

In dem fermentativen Reaktionsschritt wird Ethanol und/oder mindestens ein funktionalisierter niedermolekularer Kohlenwasserstoff hergestellt. Unter einem funktionalisierten niedermolekularen Kohlenwasserstoff K ist dabei eine lineare, verzweigte oder cyclische Verbindung mit bis zu 10 Kohlenwasserstoffatomen zu verstehen, die mindestens eine Funktionalisierung aufweisen. Unter mindestens einer Funktionalisierung ist dabei das Vorliegen mindestens einer funktionellen Gruppe zu verstehen, die eine Umsetzung des funktionalisierten Kohlenwasserstoffs zu anderen, ggf. ebenfalls funktionalisierten Kohlenwasserstoffen, insbesondere solchen mit höherem Molekulargewicht, ermöglicht. Bevorzugte funktionelle Gruppen sind Kohlenstoff-Kohlenstoff-Doppelbindungen, Kohlenstoff-Kohlenstoff-Dreifachbindungen und an Kohlenstoffatome der Kohlenwasserstoffe gebundene Hydroxy- und Carbonylgruppen. Bevorzugt handelt es sich somit bei dem mindestens einem funktionalisierten niedermolekularen Kohlenwasserstoff K um mindestens einen niedermolekularen Kohlenwasserstoff K mit mindestens einer funktionellen Gruppe ausgewählt aus der Gruppe bestehend aus Kohlenstoff-Kohlenstoff-Doppelbindungen, Kohlenstoff-Kohlenstoff-Dreifachbindungen und an Kohlenstoffatome der Kohlenwasserstoffe gebundene Hydroxy- und Carbonylgruppen. Bevorzugt weist der mindestens eine niedermolekulare Kohlenwasserstoff K zwei bis vier Kohlenstoffatome auf. Noch weiter bevorzugt ist umfasst der mindestens eine niedermolekulare Kohlenwasserstoff K mindestens eine Verbindung ausgewählt aus der Gruppe bestehend aus Acetaldehyd, Aceton, Propen, Isobuten und Butadien. Darüber hinaus noch weiter bevorzugt umfasst der mindestens eine Kohlenwasserstoff K Aceton und mindestens eine weitere Verbindung ausgewählt aus der Gruppe bestehend aus Acetaldehyd, Propen, Isobuten und Butadien.

Ganz besonders bevorzugt handelt es sich bei dem funktionalisierten niedermolekularen Kohlenwasserstoff K um Aceton.

Fermentative Verfahren zur Herstellung von Ethanol und/oder mindestens einem funktionalisierten niedermolekularen Kohlenwasserstoff K sind im Stand der Technik bekannt.

Ganz besonders bevorzugt wird im fermentativen Reaktionsschritt (III) Ethanol hergestellt. Fermentative Verfahren zur Herstellung von Ethanol offenbart z. B. US 2010/0317074 A1. Auch US 2012/0052541 A1 offenbart ein fermentatives Verfahren zur Herstellung von Ethanol. Bevorzugt einsetzbare Bakterienstämme können ausgewählt werden aus der Gruppe bestehend aus den Wildtypen von *Clostridium, Moorella* und *Acetobacterium.* Weiter bevorzugt ist der eingesetzte Bakterienstamm ein Clostridium-Wildtyp. Ganz besonders bevorzugt ist der Bakterienstamm ausgewählt aus *Clostridium autoethanogenum, Clostridium Ijungdahlii, Clostridium carboxidivorans Clostridium aceticum, Acetobacterium woodii,* und *Moorella thermoacetica.* Ausschließliche Herstellung von Ethanol kann besonders gut erzielt werden mit *Clostridium autoethanogenum.*

Ebenfalls bevorzugt wird im fermentativen Reaktionsschritt (III) Ethanol und/oder Aceton hergestellt.

Ganz besonders bevorzugt wird im fermentativen Reaktionsschritt (III) Ethanol und ein funktionalisierter niedermolekularer Kohlenwasserstoff hergestellt, da dann insgesamt, d.h. für das aus den Reaktionsschritten (I), (II) und (III) bestehende Verfahren besonders gute Ausbeuten resultieren.

Noch weiter bevorzugt wird im fermentativen Reaktionsschritt (III) Ethanol und Aceton hergestellt. Fermentative Verfahren zur Herstellung von Ethanol und Aceton sind ebenfalls im Stand der Technik bekannt. Für die Erzeugung von Aceton müssen dabei die bereits erwähnten einsetzbaren Bakterienstämme genetisch modifiziert werden. Zur Erzeugung von Ethanol und Aceton können dabei die entsprechend genetisch modifizierten Bakterienstämme allein oder zusammen mit den nicht genetisch modifizierten Bakterienstämmen eingesetzt werden. Bevorzugt wird mindestens ein Bakterienstamm eingesetzt, der ausgewählt wird aus der Gruppe bestehend aus den zur Herstellung von Ethanol und Aceton genetisch modifizierten Stämmen vom Typ *Clostridium, Moorella* und *Acetobacterium.* Weiter bevorzugt ist der mindestens eine Bakterienstamm ein zur Herstellung von Ethanol und Aceton genetisch modifizierter Clostridium-Stamm. Ganz besonders bevorzugt ist der Bakterienstamm ausgewählt aus den zur Herstellung von Ethanol und Aceton genetisch modifizierten Bakterienstämmen von *Clostridium autoethanogenum, Clostridium Ijungdahlii, Clostridium carboxidivorans, Clostridium aceticum* und *Moorella thermoacetica.*

Noch weiter bevorzugt ist der Bakterienstamm ein genetisch modifizierter Stamm bestehend aus der Gruppe aus *Clostridium Ijungdahlii, Clostridium autoethanogenum* und *Moorella thermoacetica,* da mit diesem ein Ethanol- und Aceton-reiches Produktgemisch, insbesondere ein Reaktionsgemisch bestehend aus 50 - 90 Gew.-% Aceton und 10 - 50 Gew.-% Ethanol hergestellt werden kann.

Nach der Aufreinigung des Produktgemisches aus dem fermentativen Reaktionsschritt (III), wobei bevorzugt das Produktgemisch des Schrittes (III) destillativ bei niedrigem Druck, mit einer Membran (Nanofiltration), via Pertraktion, Pervaporation, Strippen, Extraktion oder Ab-/Adsorption von der Fermentationsbrühe/dem Rückstand des eingesetzten biologischen Materials separiert wird, können die Produkte, insbesondere erhaltenes Aceton und Ethanol, voneinander und von Nebenprodukten separiert werden. Die Separation der Produkte erfolgt dabei bevorzugt destillativ, insbesondere in einer Destillationskolonne.

Entstandenes Ethanol des fermentativen Reaktionsschrittes (III) wird der chemischen Umsetzung (I) zugeführt.

Entstandener funktionalisierter niedermolekularer Kohlenwasserstoff K, insbesondere entstandenes Aceton, des fermentativen Reaktionsschrittes (III) wird mit dem Produktstrom der chemischen Umsetzung (I) vereinigt.

Besonders hohe Ausbeuten und Selektivitäten unter minimierter Nebenproduktbildung, großer Ressourceneffizienz und Verminderung des COz-Fußabdrucks können insbesondere erzielt werden, wenn
- im fermentativen Reaktionsschritt (III) sowohl Ethanol als auch niedermolekularer Kohlenwasserstoff K (bevorzugt Aceton) erzeugt,
- entstandenes Ethanol dem chemischen Reaktionsschritt (I) zugeführt und
- entstandener niedermolekularer Kohlenwasserstoff K (bevorzugt Aceton) mit dem Produktstrom der chemischen Umsetzung (I) vereinigt wird.

## Patentansprüche

1. Verfahren zur chemischen Umsetzung (I)
- von Ethanol und Wasser
- zu mindestens einem funktionalisierten niedermolekularen Kohlenwasserstoff K, Kohlendioxid und Wasserstoff,
**dadurch gekennzeichnet, dass**
mindestens ein Teil des entstehenden Kohlendioxids und/oder Wasserstoffs von dem Produktgemisch
- abgetrennt (II) und
- einem fermentativen Reaktionsschritt (III) zugeführt wird, mit dem Ethanol und/oder mindestens ein funktionalisierter niedermolekularer Kohlenwasserstoff K hergestellt wird, und nachfolgend
- ggf. entstandenes Ethanol der chemischen Umsetzung (I) zugeführt wird und
- ggf. entstandener funktionalisierter niedermolekularer Kohlenwasserstoff K mit dem Produktstrom der chemischen Umsetzung (I) vereinigt wird.

2. Verfahren nach Anspruch 1,
**dadurch gekennzeichnet, dass**
die Abtrennung (II) mindestens ein Membranseparationsverfahren einsetzt.

3. Verfahren nach Anspruch 2
**dadurch gekennzeichnet, dass**
mindestens ein Membranseparationsverfahren mindestens eine polymere Membran einsetzt.

4. Verfahren nach Anspruch 3,
**dadurch gekennzeichnet, dass**
mindestens ein Membranseparationsverfahren eine gummiartige Membran einsetzt.

5. Verfahren nach einem der vorhergehenden Ansprüche,
**dadurch gekennzeichnet, dass**
die Abtrennung (II) so durchgeführt wird, dass zunächst
i) mindestens ein Kondensationsschritt unter Erhalt eines kondensierten Kohlendioxid- und Wasserstoff-armen Produktgemischs und einer Kohlendioxid- und Wasserstoff-reichen gasförmigen Phase durchgeführt wird, und
ii) dann die erhaltene gasförmige Phase mit mindestens einem Membranseparationsverfahren unter Erhalt eines gereinigten gasförmigen Kohlendioxid-Wasserstoff-Gemischs aufgereinigt wird.

6. Verfahren nach einem der vorhergehenden Ansprüche,
**dadurch gekennzeichnet, dass**
die Umsetzung (I) von Ethanol und Wasser zu Aceton, Kohlendioxid und Wasserstoff führt.

7. Verfahren nach Anspruch 6,
**dadurch gekennzeichnet, dass**
das Verfahren ein einstufiges Verfahren ist, bei dem die Umsetzung in Gegenwart eines heterogenen Katalysators umfassend mindestens ein aktives Metall ausgewählt aus Zn, Co, Ca, Fe, Mg, Cu, Ga, Cr, Na, K, Zr, Ag, Ce, W, Al, V, La und Sr erfolgt.

8. Verfahren nach Anspruch 6,
**dadurch gekennzeichnet, dass**
das Verfahren ein zweistufiges Verfahren ist, bei dem zunächst in einem ersten Schritt
i) unter Abwesenheit von Wasser und in Gegenwart eines heterogenen Katalysators umfassend mindestens ein aktives Metall ausgewählt aus Au, Cu, Cr, Zn, Rb, Mg, Al, Fe, and V zu einem Intermediat ausgewählt aus Acetaldehyd, Crotonaldehyd, Essigsäure und Ethylacetat umgesetzt wird und dann
ii) in einem zweiten Reaktionsschritt die Umsetzung des zwischenzeitlich entstandenen Intermediats zu Aceton in Gegenwart von Wasser und einem heterogenen Katalysator umfassend mindestens ein Metall ausgewählt aus Cu, Al, Zn, and Ba erfolgt.

9. Verfahren nach einem der Ansprüche 6 - 8,
**dadurch gekennzeichnet, dass**
das molare Verhältnis von Wasser zu Ethanol im Bereich von 0,5 : 1 bis 10 : 1 liegt.

10. Verfahren nach einem der vorhergehenden Ansprüche,
**dadurch gekennzeichnet, dass**
der Feed-Strom des fermentativen Reaktionsschrittes (III)
a) aus dem in Schritt (II) abgetrennten Kohlendioxid und/oder Wasserstoff aus dem Produktgemisch des Reaktionsschrittes (I) und
b) Kohlendioxid und/oder Kohlenmonoxid aus anderen Produktionsquellen besteht.

11. Verfahren nach Anspruch 10,
**dadurch gekennzeichnet, dass**
bis zu 10 Vol.-% Kohlendioxid aus anderen Produktionsquellen und/oder bis zu 16 Vol.-% Kohlenmonoxid aus anderen Produktionsquellen, jeweils bezogen auf die eingesetzte Menge an Wasserstoff und Kohlendioxid aus dem chemischen Umsetzungsschritt (I), zugesetzt wird.

12. Verfahren nach einem der vorhergehenden Ansprüche,
**dadurch gekennzeichnet, dass**
mit dem fermentativen Reaktionsschritt (III) Ethanol hergestellt wird.

13. Verfahren nach Anspruch 12,
**dadurch gekennzeichnet, dass**
mindestens ein Bakterienstamm ausgewählt aus den Wildtypen von *Clostridium autoethanogenum, Clostridium Ijungdahlii, Clostridium carboxidivorans Clostridium aceticum, Acetobacterium woodii,* und *Moorella thermoacetica* eingesetzt wird.

14. Verfahren nach einem der Ansprüche 1 bis 11,
**dadurch gekennzeichnet, dass**
mit dem fermentativen Reaktionsschritt (III) Ethanol und Aceton hergestellt wird.

15. Verfahren nach Anspruch 14,
**dadurch gekennzeichnet, dass**
mindestens ein Bakterienstamm ausgewählt aus den zur Herstellung von Ethanol und Aceton genetisch modifizierten Bakterienstämmen von *Clostridium autoethanogenum, Clostridium Ijungdahlii, Clostridium carboxidivorans, Clostridium aceticum* und *Moorella thermoacetica* eingesetzt wird.

## Geänderte Patentansprüche

### Geänderte Patentansprüche gemäss Regel 137(2) EPÜ.

1. Verfahren zur chemischen Umsetzung (I)
- von Ethanol und Wasser
- zu Aceton, Kohlendioxid und Wasserstoff,
**dadurch gekennzeichnet, dass**
mindestens ein Teil des entstehenden Kohlendioxids und/oder Wasserstoffs von dem Produktgemisch
- abgetrennt (II) und
- einem fermentativen Reaktionsschritt (III) zugeführt wird, mit dem Ethanol und/oder mindestens ein funktionalisierter niedermolekularer Kohlenwasserstoff K hergestellt wird, und nachfolgend
- ggf. entstandenes Ethanol der chemischen Umsetzung (I) zugeführt wird und
- ggf. entstandener funktionalisierter niedermolekularer Kohlenwasserstoff K mit dem Produktstrom der chemischen Umsetzung (I) vereinigt wird.

2. Verfahren nach Anspruch 1,
**dadurch gekennzeichnet, dass**
die Abtrennung (II) mindestens ein Membranseparationsverfahren einsetzt.

3. Verfahren nach Anspruch 2
**dadurch gekennzeichnet, dass**
mindestens ein Membranseparationsverfahren mindestens eine polymere Membran einsetzt.

4. Verfahren nach Anspruch 3,
**dadurch gekennzeichnet, dass**
mindestens ein Membranseparationsverfahren eine gummiartige Membran einsetzt.

5. Verfahren nach einem der vorhergehenden Ansprüche,
**dadurch gekennzeichnet, dass**
die Abtrennung (II) so durchgeführt wird, dass zunächst
i) mindestens ein Kondensationsschritt unter Erhalt eines kondensierten Kohlendioxid- und Wasserstoff-armen Produktgemischs und einer Kohlendioxid- und Wasserstoff-reichen gasförmigen Phase durchgeführt wird, und
ii) dann die erhaltene gasförmige Phase mit mindestens einem Membranseparationsverfahren unter Erhalt eines gereinigten gasförmigen Kohlendioxid-Wasserstoff-Gemischs aufgereinigt wird.

6. Verfahren nach einem der vorhergehenden Ansprüche,
**dadurch gekennzeichnet, dass**
die chemische Umsetzung (I) einstufig und in Gegenwart eines heterogenen Katalysators umfassend mindestens ein aktives Metall ausgewählt aus Zn, Co, Ca, Fe, Mg, Cu, Ga, Cr, Na, K, Zr, Ag, Ce, W, Al, V, La und Sr erfolgt.

7. Verfahren nach einem der vorhergehenden Ansprüche,
**dadurch gekennzeichnet, dass**
die chemische Umsetzung (I) zweistufig erfolgt, wobei zunächst in einem ersten Schritt
i) unter Abwesenheit von Wasser und in Gegenwart eines heterogenen Katalysators umfassend mindestens ein aktives Metall ausgewählt aus Au, Cu, Cr, Zn, Rb, Mg, Al, Fe, and V zu einem Intermediat ausgewählt aus Acetaldehyd, Crotonaldehyd, Essigsäure und Ethylacetat umgesetzt wird und dann
ii) in einem zweiten Reaktionsschritt die Umsetzung des zwischenzeitlich entstandenen Intermediats zu Aceton in Gegenwart von Wasser und einem heterogenen Katalysator umfassend mindestens ein Metall ausgewählt aus Cu, Al, Zn, and Ba erfolgt.

8. Verfahren nach einem der vorhergehenden Ansprüche,
**dadurch gekennzeichnet, dass**
das molare Verhältnis von Wasser zu Ethanol im Bereich von 0,5 : 1 bis 10 : 1 liegt.

9. Verfahren nach einem der vorhergehenden Ansprüche,
**dadurch gekennzeichnet, dass**
der Feed-Strom des fermentativen Reaktionsschrittes (III)
a) aus dem in Schritt (II) abgetrennten Kohlendioxid und/oder Wasserstoff aus dem Produktgemisch des Reaktionsschrittes (I) und
b) Kohlendioxid und/oder Kohlenmonoxid aus anderen Produktionsquellen besteht.

10. Verfahren nach Anspruch 9,
**dadurch gekennzeichnet, dass**
bis zu 10 Vol.-% Kohlendioxid aus anderen Produktionsquellen und/oder bis zu 16 Vol.-% Kohlenmonoxid aus anderen Produktionsquellen, jeweils bezogen auf die eingesetzte Menge an Wasserstoff und Kohlendioxid aus dem chemischen Umsetzungsschritt (I), zugesetzt wird.

11. Verfahren nach einem der vorhergehenden Ansprüche,
**dadurch gekennzeichnet, dass**
mit dem fermentativen Reaktionsschritt (III) Ethanol hergestellt wird.

12. Verfahren nach Anspruch 11,
**dadurch gekennzeichnet, dass**
mindestens ein Bakterienstamm ausgewählt aus den Wildtypen von *Clostridium autoethanogenum, Clostridium Ijungdahlii, Clostridium carboxidivorans Clostridium aceticum, Acetobacterium woodii,* und *Moorella thermoacetica* eingesetzt wird.

13. Verfahren nach einem der Ansprüche 1 bis 10,
**dadurch gekennzeichnet, dass**
mit dem fermentativen Reaktionsschritt (III) Ethanol und Aceton hergestellt wird.

14. Verfahren nach Anspruch 13,
**dadurch gekennzeichnet, dass**
mindestens ein Bakterienstamm ausgewählt aus den zur Herstellung von Ethanol und Aceton genetisch modifizierten Bakterienstämmen von *Clostridium autoethanogenum, Clostridium Ijungdahlii, Clostridium carboxidivorans, Clostridium aceticum* und *Moorella thermoacetica* eingesetzt wird.
